# EUROPÄISCHE PATENTSCHRIFT

(11) **EP 0 381 962 B1**
(45) Veröffentlichungstag und Bekanntmachung des Hinweises auf die Patenterteilung: **08.06.1994**
(21) Anmeldenummer: 90100982.9
(22) Anmeldetag: 18.01.1990
(51) Int. Cl.: A61B 17/54

(54) **Elektrischer Hornhautschleifer**
Electrical callous abrading instrument
Appareil électrique de ponçage des durillons

(30) Priorität: 09.02.1989 DE 3903828
(43) Veröffentlichungstag der Anmeldung: 16.08.1990
(73) Patentinhaber: PETER BAUSCH GMBH & CO. KG., 42369 Wuppertal (DE)
(72) Erfinder: Schnitzler, Wolfgang, D-5609 Bergisch Born (DE); Scaglione, Gerado, D-5600 Wuppertal 21 (DE); Gottschalk, Werner, D-5630 Remscheid 11 (DE)
(74) Vertreter: Peerbooms, Rudolf, Dipl.-Phys.

(56) Entgegenhaltungen:
- DE-A- 1 457 446
- FR-A- 1 453 515
- FR-A- 2 383 751
- GB-A- 2 092 003

## Beschreibung

Die Erfindung betrifft einen elektrischen Hornhautschleifer, mit in einem länglichen Handgriff angeordnetem Antriebsmotor, mit dessen aus dem vorderen Handgriffende herausragender Antriebswelle ein Schleifwerkzeug antriebsmäßig verbunden ist.

Durch die DE-OS 37 40 902 und das DE-GM 85 27 068 sind elektrische Hornhautschleifer bekannt, bei denen das spanabhebende Schleifwerkzeug aus einer Raspelrolle besteht, die nur auf einem Teil ihres Umfanges freiliegt und im übrigen zur Vermeidung einer Verletzungsgefahr durch verlängerte Gehäusewände abgedeckt ist. Bei solchen Geräten mit rotierenden Raspelrollen oder bei Fräswerkzeuggeräten zum Entfernen von Hornhaut, wie diese beispielsweise im DE-GM 81 32 041 oder in der DE-AS 12 08 448 beschrieben sind, besteht trotzdem bei unsachgemäßer Handhabung die Gefahr einer Verletzung durch überstarken Hornhautabtrag. Darüber hinaus sind die bekannten Geräte in hygienischer Hinsicht kritisch, da sie aufgrund ihrer Abdeckungen nur schwierig sauberzuhalten und zu desinfizieren sind. Bei dem professionellen Einsatz besteht die große Gefahr einer Übertragung von Infektionskrankheiten, beispielsweise von Fußpilz.

Der Erfindung liegt die Aufgabe zugrunde, einen schonend arbeitenden und jegliche Verletzungsgefahr ausschließenden Hornhautschleifer zu schaffen, der sowohl für den Selbstanwender aufgrund einfachster Handhabung, Wartung und niedrigen Preises geeignet ist und der gleichermaßen für einen professionellen Einsatz unter voller Berücksichtigung der Hygieneprobleme verwendbar sein soll.

Zur Lösung dieser Aufgabe ist erfindungsgemäß vorgesehen, daß das Schleifwerkzeug aus einer gummielastischen, am Außenboden einen Schleifbelag aufweisenden Kappe besteht, die mit ihrem offenen Ende auf das vordere Handgriffende abnehmbar aufgesetzt ist und an ihrem Innenboden eine exzentrische Aufnahme für einen exzentrischen Endabschnitt der Antriebswelle aufweist.

Durch die Erfindung werden eine Reihe von Vorteilen erreicht. Zum einen wird der gesamte Antriebswellenbereich staubdicht - und bei strammer Passung sogar wasserdicht - durch die elastische Kappe abgedeckt, so daß abgeriebene Hornhautteilchen höchstens bis zur Außenfläche des Handgriffteiles gelangen können, der aufgrund seiner glatten Oberfläche leicht sauberzuhalten ist, was ebenfalls für den glatten Mantel der Kappe gilt. Bei dem als Schwingschleifer ausgebildeten Gerät führt der Schleifbelag eine verhältnismäßig kurze hin- und hergehende Bewegung aus, mit der feinfühlig Hornhaut abgetragen werden kann, ohne daß die Gefahr einer Verletzung besteht.

Nach einem weiteren Merkmal der Erfindung ist die Kappe an ihrer äußeren Bodenfläche mit einer Klettenverschluß-Scheibe und mit daran auswechselbar befestigten Schleifpapier-Scheiben versehen. Nach Abnutzung einer Schleifscheibe kann diese gegen eine neue ausgetauscht werden. Ebenfalls ist es möglich, dem Gerät einen Satz von Schleifpapierscheiben unterschiedlicher Körnung zuzuordnen, die nach dem jeweiligen Anwendungsfall ausgewählt werden.

Die elastische Kappe, die der Erfindung zufolge um mehrere Fingerbreiten den Handgriff überragen soll, weist eine hohe Festigkeit gegenüber der Schwingbewegungen auf und kann somit über lange Betriebszeiten im Einsatz bleiben, was für einen Selbstanwender von großem Vorteil ist.

Andererseits stellt aber die Kunststoffkappe mit angebrachtem Klettenverschluß ein verhältnismäßig billiges Bauelement dar, das dem professionellen Fußpfleger in größeren Stückzahlen, jeweils in sterilen Einzelabpackungen geliefert werden kann, so daß dieser für jede zu behandelnde Person eine neue Kappe einsetzen kann, womit jeglicherAnsteckungsgefahr vorgebeugt ist. Das Aufstecken und Abziehen der Kappe ist dabei mit wenigen Handgriffen durchführbar, insbesondere wenn das vordere Ende des Handgriffes etwas konisch eingezogen ist. Dabei kann der Halt der Kappe noch dadurch gesichert werden, daß diese mit einem verstärkten Schnapprand und der Handgriff mit einer zugeordneten, umlaufenden Schnappnut versehen ist.

Weitere Merkmale der Erfindung sind in den Unteransprüchen angegeben.

Der Gegenstand der Erfindung wird im folgenden anhand dreier in der Zeichnung dargestellter Ausführungsbeispiele näher beschrieben. In der Zeichnung zeigen:
- Fig. 1: ein erstes Ausführungsbeispiel des Hornhautschleifers nach der Erfindung, teils in Draufsicht, teils im Längsschnitt,
- Fig. 2: einen Schnitt gemäß der Schnittebene II-II in Fig. 1,
- Fig. 3: ein zweites Ausführungsbeispiel des Hornhautschleifers nach der Erfindung,
- Fig. 4: einen Schnitt gemäß der Linie IV-IV in Fig. 3 und
- Fig. 5: ein drittes Ausführungsbeispiel des Hornhautschleifers nach der Erfindung.

Der Hornhautschleifer besitzt ein als stabförmigen Handgriff 1 ausgebildetes Gehäuse für einen Motor (nicht gezeigt), welcher beispielsweise über das Kabel 2 an ein Regelnetzgerät anschließbar und über den Schalter 3 einschaltbar ist. Aus dem vorderen Ende 4 des Handgriffes 1 ragt stirnseitig eine Antriebswelle 5 für ein Schleifwerkzeug 6 heraus. Das Schleifwerkzeug 6 besteht aus einer gummielastischen Kappe 7, die mit ihrem offenen Ende auf das Handgriffende 4 in strammem Sitz aufgeschoben ist. Auf ihren Außenboden ist eine Klettenverschluß-Scheibe 8 aufgeklebt und darauf eine Schleifpapierscheibe 9 aufgedrückt.

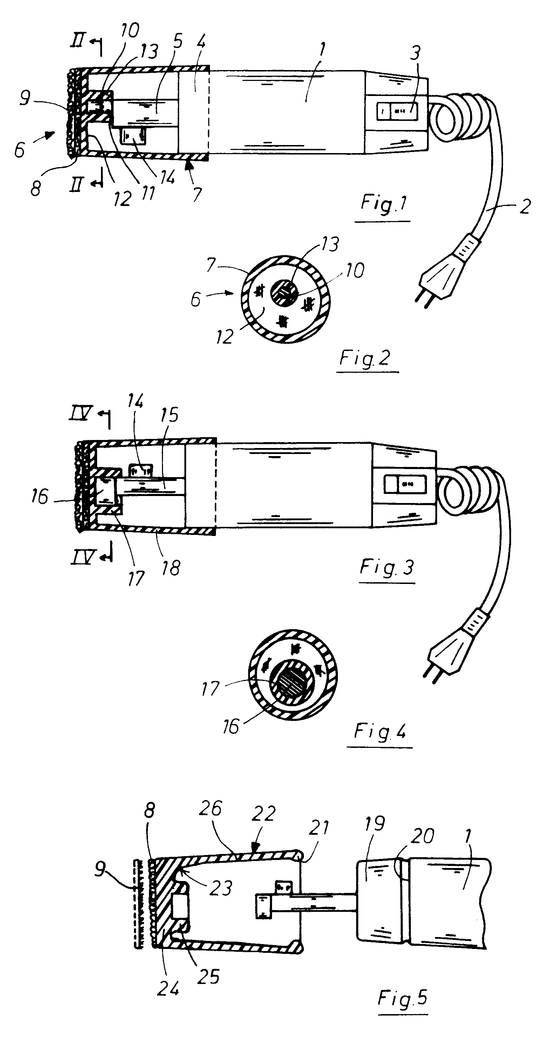

Die Antriebswelle 5 endet beim Ausführungsbeispiel nach den Fig. 1 und 2 in einem im Durchmesser verjüngten, exzentrischen Stift 10, der in eine exzentrische Lageraufnahme 11 am Innenboden 12 der Kappe 7 eingreift. Die Lageraufnahme 11 ist durch eine exzentrische, angeformte Buchse 13 gebildet. An einer dem Exzenterstift diametral gegenüberliegenden, einwärts versetzten Stelle trägt die Antriebswelle 5 eine Unwuchtmasse 14. Nach Einschalten arbeitet das Gerät als ein Schwingschleifer, bei dem die Schleifpapierscheibe 9 innerhalb einer Ebene translatorisch auf einer Kreisbahn bewegt wird.

Beim Ausführungsbeispiel nach den Fig. 3 und 4 ist im Unterschied zum ersten Ausführungsbeispiel die Antriebswelle 15 mit einem kleineren Durchmesser versehen als ihr exzentrisches Ende 16, für dessen Aufnahme eine entsprechend größere Buchse 17 am Boden der Kappe 18 vorgesehen ist.

Beim Ausführungsbeispiel nach Fig. 5 ist das vordere Handgriffende 19 leicht kegelig eingezogen und mit einer umlaufenden Schnappnut 20 für die einen verstärkten Schnapprand 21 aufweisende Kappe 22 versehen. Der Mantel der Kappe 22 ist leicht konisch ausgebildet und geht über einen abgerundeten Bereich 23 in den Kappenboden 24 über, wodurch eine bessere Verteilung der Wechselbelastung innerhalb der Kappe erreicht wird. Wie insbesondere beim Ausführungsbeispiel 5 gezeigt ist, ist die Wandstärke von Kappenboden 24 und Buchse 25 deutlich größer als die Wandstärke des Kappenmantels 26.

## Patentansprüche

1. Elektrischer Hornhautschleifer, mit in einem länglichen Handgriff angeordnetem Antriebsmotor, mit dessen aus dem vorderen Handgriffende herausragender Antriebswelle ein Schleifwerkzeug antriebsmäßig verbunden ist, dadurch gekennzeichnet, daß das Schleifwerkzeug (6) aus einer gummielastischen, am Außenboden einen Schleifbelag (9) aufweisenden Kappe (7) besteht, die mit ihrem offenen Ende auf das vordere Handgriffende (4) abnehmbar aufgesetzt ist und an ihrem Innenboden (12) eine exzentrische Aufnahme (11) für einen exzentrischen Endabschnitt (10) der Antriebswelle (5) aufweist.

2. Hornhautschleifer nach Anspruch 1, dadurch gekennzeichnet, daß die Kappe (7) an ihrer äußeren Bodenfläche eine Klettenverschluß-Scheibe (8) und eine daran auswechselbar befestigte Schleifpapierscheibe (9) trägt.

3. Hornhautschleifer nach den Ansprüchen 1 und 2, dadurch gekennzeichnet, daß der Handgriff (1) als runder Stab ausgebildet ist, der an seinem vorderen Ende (19) leicht kegelig eingezogen ist.

4. Hornhautschleifer nach Anspruch 3, dadurch gekennzeichnet, daß die Kappe (7) einen im wesentlichen zylindrischen Mantel besitzt, und daß der Mantel und die Antriebswelle (5) den Handgriff (1, 4) um etwa zwei bis vier Fingerbreiten überragen.

5. Hornhautschleifer nach einem der vorangegangenen Ansprüche, dadurch gekennzeichnet, daß der Kappenboden (24) und die die exzentrische Bohrung umschließende angeformte Buchse (25) eine größere Wandstärke als der Kappenmantel (26) aufweisen.

6. Hornhautschleifer nach Anspruch 5, dadurch gekennzeichnet, daß der Kappeninnenboden (24) über eine Abrundung (23) in den Mantel (26) übergeht.

7. Hornhautschleifer nach einem der Ansprüche 1 bis 6, dadurch gekennzeichnet, daß die Kappe (22) mit einem verstärkten Schnapprand (21) und der Handgriff mit einer zugeordneten, umlaufenden Schnappnut (20) versehen ist.

8. Hornhautschleifer nach einem der vorangegangenen Ansprüche 1 bis 7, dadurch gekennzeichnet, daß die Antriebswelle (5) eine Unwuchtmasse (14) trägt.

## Claims

1. Electric callous abrading instrument, with a motor-drive, set in a longitudinal handle, to whose drive-shaft, projecting from the front end of the handle, an abrasion tool is connected in line with the drive, characterized in that the abrasion tool (6) consists of an elastic rubber hood (7) with an abrasive coating (9) on the exterior surface of its closed top end and with its open end placed, removably, upon the front end (4) of the handle and having an eccentric socket (11) on the interior surface (12) of its top end to accommodate an eccentric end-section (10) of the drive-shaft (5).

2. Callous abrading instrument as claimed in Claim 1, characterized in that the hood (7) has a disc of hook-and-loop fastening material (8) on its exterior top surface with a interchangeable abrasive-paper disc (9) applied to it.

3. Callous abrading instrument as claimed in Claims 1 and 2, characterized in that the handle (1) is designed as a round rod whose front end (19) is slightly narrowed into a conical shape.

4. Callous abrading instrument as claimed in Claim 3, characterized in that the hood (7) has an essentially cylindrical sleeve and that the sleeve and drive shaft (5) project beyond the handle (1,4) by approximately two to four finger-breadths.

5. Callous abrading instrument as claimed in one of the preceding claims, characterized in that the top (24) of the hood and the socket (25) moulded into it and surrounding the eccentric bore have thicker walls than the sleeve (26) of the hood.

6. Callous abrading instrument as claimed in Claim 5, characterized in that the interior surface of the top (24) of the hood passes via a rounded-off area (23) into the sleeve (26).

7. Callous abrading instrument as claimed in one of Claims 1 to 6, characterized in that the hood (22) is equipped with a reinforced snap-fastening edge (21) and the handle has a corresponding snap-fastening groove (20) running round it.

8. Callous abrading instrument as claimed in one of the previous Claims 1 to 7, characterized in that the drive shaft (5) carries an unbalanced mass (14).

## Revendications

1. Appareil électrique à meuler les callosités, avec un moteur d'entraînement disposé dans une poignée allongée et à l'arbre d'entraînement, ressortant de l'extrémité avant de la poignée, duquel un outil de meulage est relié en entraînement, caractérisé en ce que l'outil de meulage (6) est composé d'un capuchon (7), ayant l'élasticité du caoutchouc, présentant une garniture de meulage (9) et appliqué par son extrémité ouverte sur l'extrémité avant (4) de la poignée et présente sur son fond intérieur (12) un logement (11) excentrique, pour un tronçon d'extrémité (10) excentrique de l'arbre d'entraînement (5).

2. Appareil à meuler les callosités selon la revendication 1, caractérisé en ce que le capuchon (7) porte, sur sa surface de fond extérieure, un disque de fermeture auto-accrochant (8) et un disque de papier abrasif (9), fixé dessus remplaçable.

3. Appareil à meuler les callosités selon les revendications 1 et 2, caractérisé en ce que la poignée (1) est réalisée sous forme de barre à section ronde, légèrement rétreinte de façon conique à son extrémité avant (19).

4. Appareil à meuler les callosités selon la revendication 3, caractérisé en ce que le capuchon (7) comporte une enveloppe sensiblement cylindrique, et en ce que l'enveloppe et l'arbre d'entraînement (5) dépassent de la poignée (1, 4) d'à peu près deux à quatre fois la largeur d'un doigt.

5. Appareil à meuler les callosités selon l'une des revendications précédentes, caractérisé en ce que le fond de capuchon (24) et la douille (25), formée d'un seul tenant et entourant le perçage excentrique, présentent une épaisseur de paroi supérieure à celle de l'enveloppe de capuchon (26).

6. Appareil à meuler les callosités selon la revendication 5, caractérisé en ce que le fond intérieur de capuchon (24) se transforme en l'enveloppe (26), par l'intermédiaire d'un arrondi (23).

7. Appareil à meuler les callosités selon l'une des revendications 1 à 6, caractérisé en ce que le capuchon (22) est pourvu d'une bordure d'encliquetage (21) à épaisseur renforcée et la poignée est pourvue d'une gorge d'encliquetage (20) associée, faisant le pourtour.

8. Appareil à meuler les callosités selon l'une des revendications 1 à 7 précédentes, caractérisé en ce que l'arbre d'entraînement (5) porte une masse de balourd (14).
